# EUROPEAN PATENT APPLICATION

(11) **EP 4 537 837 A1**
(43) Date de publication de la demande: **16.04.2025**
(21) Numéro de dépôt: 22945256.0
(22) Date de dépôt: 08.06.2022
(51) Int. Cl.: A61K 36/04, A61P 31/00, A61Q 1/00, A61Q 7/00

(54) **PROCÉDÉ D'EXTRACTION DE SARCODIA MONTAGNEANA, EXTRAIT DE SARCODIA MONTAGNEANA ET UTILISATION ASSOCIÉE**

(71) Demandeur: CPC Corporation, Taiwan, Kaohsiung City 81126 (TW)
(72) Inventeur: CHENG, Shu-yu, Kaohsiung City Taiwan 81126 (TW); KAO, Ai-ling, Kaohsiung City Taiwan 81126 (TW); WENG, Yu-xiang, Kaohsiung City Taiwan 81126 (TW); HUANG, Tung-li, Kaohsiung City Taiwan 81126 (TW); CHEN, Chin-chung, Kaohsiung City Taiwan 81126 (TW); HUNG, Pei-ching, Kaohsiung City Taiwan 81126 (TW); WANG, Chao-chun, Kaohsiung City Taiwan 81126 (TW); CHANG, Wen-teng, Kaohsiung City Taiwan 81126 (TW)
(74) Mandataire: Cabinet Chaillot
(86) Numéro de dépôt international: PCT/CN2022/097673
(87) Numéro de publication internationale: WO 2023/236111

(57) **Abrégé**

La présente invention concerne un procédé d'extraction de Sarcodia suiae, des extraits de Sarcodia suiae et leurs utilisations associées, lesdits extraits de Sarcodia suiae sont obtenus et préparés selon le procédé d'extraction comprenant les étapes suivantes : (a) fourniture d'une poudre séchée de Sarcodia suiae ; (b) obtention d'un liquide d'extraction de Sarcodia suiae par extraction de ladite poudre séchée de Sarcodia suiae par fluides de dioxyde de carbone, ledit liquide d'extraction de Sarcodia suiae contenant des extraits de Sarcodia suiae et ledit solvant de fluides de dioxyde de carbone, la pression d'extraction étant de 300-650 bars absolus, la température d'extraction étant de 45-55°C, la durée d'extraction étant de 2-6 h ; et (c) séparation des extraits de Sarcodia suiae dudit liquide d'extraction de Sarcodia suiae, la pression de séparation étant de 30-100 bars absolus. Lesdits extraits de Sarcodia suiae peuvent être utilisés à la préparation de médicaments de stimulation de la croissance des cellules du follicule pileux, de médicaments ou produits cosmétiques inhibiteurs de croissance des bactéries de l'acné ou de médicaments ou produits cosmétiques de stimulation de l'activité des kératinocytes. Grâce aux extraits de Sarcodia suiae susmentionnés de nouvelles utilisations de la Sarcodia suiae à plus haute valeur économique peuvent être développées.

## Description

La présente invention concerne un procédé d'extraction de Sarcodia suiae, des extraits de Sarcodia suiae et leurs utilisations associées, et en particulier des extraits de Sarcodia suiae préparés par extraction par fluides supercritiques.

### Art antérieur

La Sarcodia suiae est une algue plate de couleur rouge sombre, la Sarcodia suiae dont nous avons connaissance actuellement est riche en fibres alimentaires, en minéraux, en aminoacides nécessaires au corps humain, en vitamine E et en acides gras oméga. C'est pourquoi, la Sarcodia suiae est considérée comme un produit alimentaire de santé.

### Résumé de l'invention

Pourtant, de nos jours, outre la Sarcodia suiae déjà connue comme produit alimentaire, il n'existe pas de procédé permettant d'extraire efficacement ses ingrédients à haute valeur économique. C'est pourquoi la fourniture d'un nouveau procédé d'extraction efficace des ingrédients à haute valeur économique de la Sarcodia suiae reste un problème à résoudre.

Visant à résoudre le problème susmentionné, la présente invention concerne un procédé d'extraction de Sarcodia suiae, comprenant les étapes suivantes : (a) fourniture d'une poudre séchée de Sarcodia suiae ; (b) extraction de ladite poudre séchée de Sarcodia suiae par fluides de dioxyde de carbone afin d'obtenir le liquide d'extraction de Sarcodia suiae, ledit liquide d'extraction de Sarcodia suiae contenant des extraits de Sarcodia suiae et ledit solvant de fluides de dioxyde de carbone, la pression d'extraction étant de 300-650 bars absolus, la température d'extraction étant de 45-55°C, la durée d'extraction étant de 2-6 h ; et (c) séparation des extraits de Sarcodia suiae dudit liquide d'extraction de Sarcodia suiae, la pression de séparation étant de 30-100 bars absolus. Et les extraits de Sarcodia suiae préparés grâce au procédé d'extraction susmentionné.

La pression d'extraction peut être de 350 bars absolus, la température d'extraction, de 55°C, la durée d'extraction, de 2 h.

La pression d'extraction peut être de 365 bars absolus ou de 420 bars absolus, la température d'extraction, de 55°C, la durée d'extraction, de 4 h.

La présente invention concerne un procédé de préparation de médicaments de stimulation de croissance du follicule pileux utilisant les extraits de Sarcodia suiae susmentionnés selon une concentration de plus de 5 µg/mL. La présente invention fournit un procédé de préparation de médicaments ou produits cosmétiques inhibiteurs de croissance des bactéries de l'acné utilisant les extraits de Sarcodia suiae susmentionnés selon une concentration de plus de 0,5 mg/mL.

La présente invention concerne un procédé de préparation de médicaments ou produits cosmétiques renforçant l'activité de migration des cellules de kératinocytes utilisant les extraits de Sarcodia suiae susmentionnés selon une concentration de plus de 1 µg/mL ou de plus de 6 µg/mL.

La présente invention concerne un nouveau procédé d'extraction pour extraire les ingrédients à haute valeur économique de la Sarcodia suiae.

### Brève description des figures

Les Figures 1 à 3 représentent l'empreinte HPLC des extraits de Sarcodia suiae du mode de réalisation 1 de la présente invention sous rayonnement UV à longueurs d'ondes respectives de 210 nm, 254 nm et 280 nm.
La figure 4 représente le spectre chromatographique en phase gazeuse des extraits de Sarcodia suiae du mode de réalisation 1 de la présente invention.
La Figure 5 à la Figure 7 représentent les résultats respectifs des essais de stimulation de croissance cellulaire des cellules de la papille dermique du follicule pileux humain par la Sarcodia suiae des modes de réalisation 1, 2 et 3 de la présente invention.
La Figure 8 représente les résultats comparatifs des empreintes HPLC de différents extraits de Sarcodia suiae du mode de réalisation 4 de la présente invention.
La Figure 9 et la Figure 10 représentent respectivement l'imagerie par fluorescence et le taux de migration cellulaire des essais de stimulation de migration des kératinocytes par la Sarcodia suiae du mode de réalisation 6 de la présente invention.
La Figure 11 représente les résultats de stimulation de croissance des kératinocytes par la Sarcodia suiae du mode de réalisation 7 de la présente invention.

### Modes de réalisation

Le procédé d'extraction de la Sarcodia suiae du mode de réalisation 1 est le suivant :
De la poudre séchée de Sarcodia suiae est d'abord fournie. Ladite poudre séchée de Sarcodia suiae est une poudre obtenue selon un mode de préparation ordinaire ou acquise par achat commercial. Le mode de préparation ordinaire de la poudre de Sarcodia suiae est le suivant, la Sarcodia suiae obtenue subit un traitement de lavage puis de séchage, le traitement de séchage consistant à placer la Sarcodia suiae dans un four de séchage à 50°C pour séchage continu pendant 24 heures, la Sarcodia suiae séchée obtenue est moulue en poudre dans un broyeur, la poudre de Sarcodia suiae séchée est ainsi obtenue, son diamètre de grain est de 1-4 mm, ce diamètre peut également être ajusté.

Puis, l'extracteur par fluides supercritiques est préparé, l'extracteur par fluides supercritiques susmentionné est fourni par le Centre de recherche et développement industriel des métaux (MIRDC), numéro de modèle SFE-350S-1000R. L'extracteur par fluides supercritiques est utilisé pour extraire des extraits de Sarcodia suiae à partir de la poudre de Sarcodia suiae susmentionnée. Le mode d'extraction est tel que décrit ci-après, la poudre de Sarcodia suiae susmentionnée est placée dans le bac d'extraction de l'extracteur par fluides supercritiques, les quantités de poudre de Sarcodia suiae utilisées représentent environ 90-99% du volume du bac d'extraction, par exemple le volume d'alimentation en poudre de Sarcodia suiae est de 850 g à 950 g, et le volume du bac d'extraction est de 1,2 L. Les fluides supercritiques sont ensuite utilisés comme solvant pour procéder à l'extraction de ladite poudre de Sarcodia suiae. Dans le présent mode de réalisation 1, les fluides supercritiques utilisés sont des fluides de dioxyde de carbone, la pression d'extraction est de 350 bars absolus, et peut être ajustée entre 300 bars absolus et 650 bars absolus selon les besoins, la température d'extraction est de 55°C, et peut être ajustée entre 45°C et 55°C selon les besoins, la durée d'extraction est de 2 heures, le débit de fluides de dioxyde de carbone est de 10 L/h, et peut être ajusté entre 5 et 15 L/h selon les besoins. Une fois l'extraction achevée un liquide d'extraction de Sarcodia suiae est obtenu, ledit liquide d'extraction de Sarcodia suiae comprenant les extraits de Sarcodia suiae et le solvant de fluides de dioxyde de carbone, en outre ledit liquide d'extraction de Sarcodia suiae est dissous dans ledit solvant de fluides de dioxyde de carbone.

Enfin, ledit liquide d'extraction de Sarcodia suiae est placé dans le bac séparateur de l'extracteur par fluides supercritiques pour réduction de pression, la pression de séparation est de 35 bars absolus, et peut être ajustée entre 30 et 100 bars absolus selon les besoins, de cette façon les extraits de Sarcodia suiae sont séparés du liquide d'extraction de Sarcodia suiae, une fois lesdits extraits de Sarcodia suiae séparés du solvant de fluides de dioxyde de carbone, les conduites d'extraction de l'extracteur à fluides supercritiques sont rincées à l'éthanol afin de purger les conduites d'extraction desdits extraits de Sarcodia suiae qui y ont adhéré, c'est-à-dire que ces extraits de Sarcodia suiae sont dissous dans l'éthanol, et l'éthanol contenant lesdits extraits dissous est évacué des conduites d'extraction, les extraits de Sarcodia suiae sont ainsi collectés, les extraits de Sarcodia suiae dissous dans l'éthanol sont placés à température ambiante afin que l'éthanol s'évapore entièrement, les extraits de Sarcodia suiae sont ainsi obtenus.

### Analyse HPLC des extraits de Sarcodia suiae du mode de réalisation 1

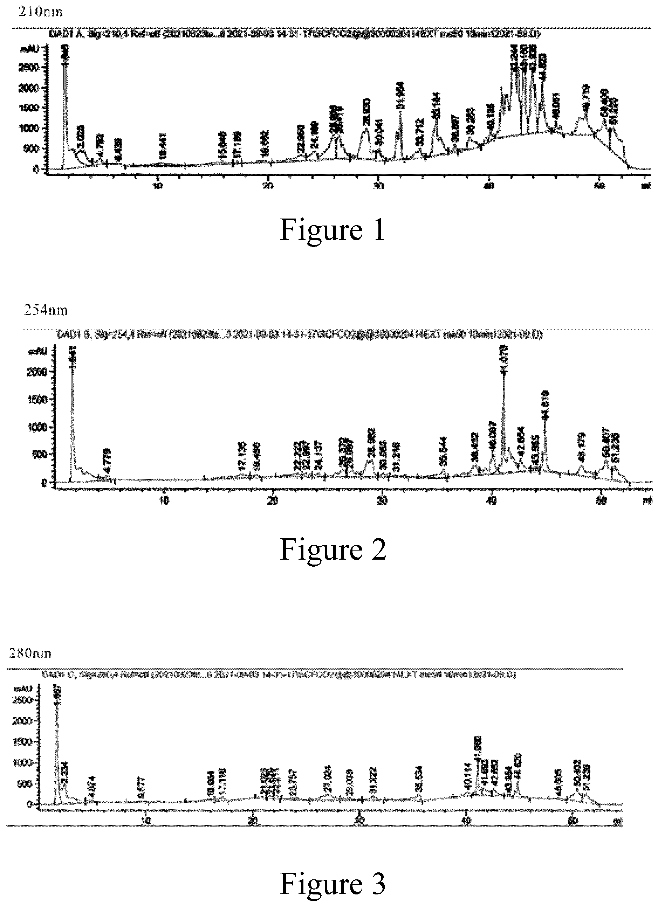

Afin d'analyser les ingrédients des extraits de Sarcodia suiae du mode de réalisation 1, une analyse sur colonnes HPLC est réalisée, les conditions d'analyse étant les suivantes, des colonnes HPLC de HPH-C188 (4,6 × 100 mm × 2,7 µm) sont utilisées, débit de 1 ml/min, une détection à l'aide d'un détecteur à ultraviolets/lumière visible Agilent 1260 DAD est effectuée, la lumière détectée est celle d'UV de longueur d'onde de 210 nm, 254 nm et 280 nm. Les figures 1-3 représentent les empreintes HPLC des extraits de Sarcodia suiae.

### Analyse GC/MS des extraits de Sarcodia suiae du mode de réalisation 1

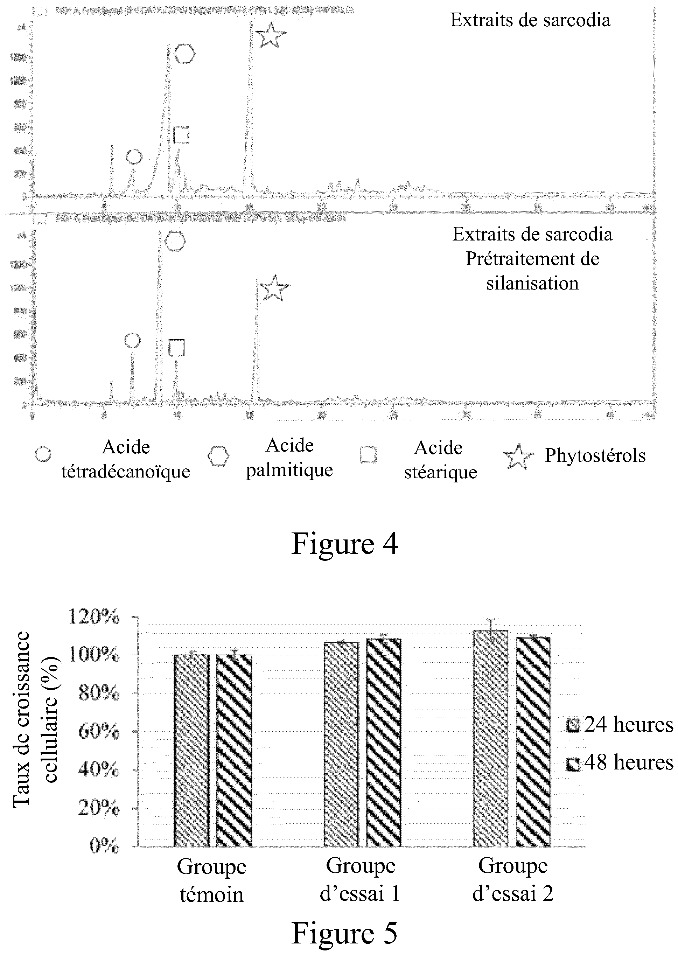

Les extraits de Sarcodia suiae susmentionnés sont placés dans le chromatographe à phase gazeuse (GC) (Agilent 7890 GC/5977B EI à détecteur par spectrométrie de masse) pour analyse, afin d'obtenir un spectre GC plus clair, un autre échantillon des extraits de Sarcodia suiae susmentionné est prélevé et subit un pré-traitement de silanisation pour réaliser une analyse de chromatographie en phase gazeuse sous les mêmes conditions. Les colonnes d'analyse utilisées pour la chromatographie en phase gazeuse susmentionnée sont des colonnes ZB-1XT SIMDIST (5 m × 0,53 mm ID, 0,09 µm d'épaisseur de membrane mince), les résultats d'analyse de chromatographie en phase gazeuse sont représentés à la Figure 4. Lesdits extraits de Sarcodia suiae contiennent des acides gras et des phytostérols tels que l'acide tétradécanoïque, l'acide palmitique et l'acide stéarique.

### Essais de stimulation de croissance cellulaire des cellules de la papille dermique du follicule pileux humain par la Sarcodia suiae du mode de réalisation 1

Des extraits de Sarcodia suiae sont d'abord préparés à l'aide du procédé susmentionné ; parallèlement, des souches HFDPC de cellules de la papille dermique du follicule pileux humain sont préparées (acquises auprès de la société PromoCell, modèle de produit C12071).

Une plaque de culture cellulaire à 96 puits est ensuite préparée, dans laquelle un liquide cellulaire contenant des cellules HFDPC est injecté à 9 puits (milieu de culture cellulaire de la papille dermique du follicule pileux, PromoCell C-26501), chaque puit comprenant 5-8 × 10³ cellules, un groupe comprenant trois puits, les 9 puits auxquels le liquide cellulaire HFDPC a été ajouté étant divisés en groupe témoin, groupe d'essai 1 et groupe d'essai 2, le groupe témoin ne contient pas d'extraits de Sarcodia suiae, une concentration finale de 5 µg/mL d'extraits de Sarcodia suiae est injectée au groupe d'essai 1 et une concentration finale de 25 µg/mL d'extraits de Sarcodia suiae est injectée au groupe d'essai 2. Une fois que des extraits de Sarcodia suiae ont été injectés au groupe 1 et au groupe 2, la plaque de culture cellulaire est placée dans un incubateur cellulaire pour culture dans un environnement à 37°C durant 48 heures, puis des échantillons sont prélevés de chaque puit à respectivement 24 h et 48 h de culture, les taux de croissance cellulaire des groupes d'essai 1 et 2 sont mesurés par essai de taux de survie cellulaire (essai MTT), le mode de calcul étant (valeur d'absorption lumineuse du groupe d'essai (1 ou 2)/valeur d'absorption lumineuse du groupe témoin) × 100.

Les résultats des essais sont représentés au Tableau 1 et à la Figure 5. À 24 h de culture, le taux de croissance cellulaire du groupe d'essai 1 par rapport au groupe témoin est de 106,5%, à 48 h de culture du groupe d'essai 1, le taux de croissance cellulaire par rapport au groupe témoin est de 108,3% ; à 24 h de culture, le taux de croissance cellulaire du groupe d'essai 2 par rapport au groupe témoin est de 113,1%, à 48 h de culture du groupe d'essai 2, le taux de croissance cellulaire par rapport au groupe témoin est de 109,2%.

**Tableau 1 : Valeurs moyennes d'absorption lumineuse du groupe témoin, du groupe d'essai 1 et du groupe d'essai 2**

| | Groupe témoin OD490 | Groupe d' essai 1 OD490 | Groupe d'essai 2 OD490 |
|---|---|---|---|
| 24 heures | 1,2 | 1,278 | 1,358 |
| 48 heures | 1,419 | 1,537 | 1,55 |

### Essais de stimulation de croissance cellulaire des cellules de la papille dermique du follicule pileux humain par la Sarcodia suiae du mode de réalisation 2

Des extraits de Sarcodia suiae sont d'abord préparés selon le procédé au groupe d'essai 1, cependant la pression d'extraction est ajustée à 365 bars absolus pour préparer les extraits de Sarcodia suiae du mode de réalisation 2 ; parallèlement, des souches HFDPC de cellules de la papille dermique du follicule pileux humain sont préparées (acquises auprès de la société PromoCell, modèle de produit C12071) .

Une plaque de culture cellulaire est ensuite préparée, dans laquelle un liquide cellulaire contenant des cellules HFDPC est injecté à 9 puits, un groupe comprenant trois puits, les 9 puits auxquels le liquide cellulaire HFDPC susmentionné a été ajouté constituant le groupe témoin, le groupe d'essai 1 et le groupe d'essai 2, le groupe témoin ne contient pas d'extraits de Sarcodia suiae, une concentration finale de 5 µg/mL d'extraits de Sarcodia suiae est injectée au groupe d'essai 1 et une concentration finale de 25 µg/mL d'extraits de Sarcodia suiae est injectée au groupe d'essai 2. Une fois que des extraits de Sarcodia suiae ont été injectés au groupe 1 et au groupe 2, la plaque de culture cellulaire est placée dans un incubateur cellulaire pour culture dans un environnement à 37°C durant 48 heures, puis des échantillons sont prélevés de chaque puits à respectivement 24 h et 48 h de culture, les taux de croissance cellulaire sont mesurés par essai de taux de survie cellulaire (essai MTT).

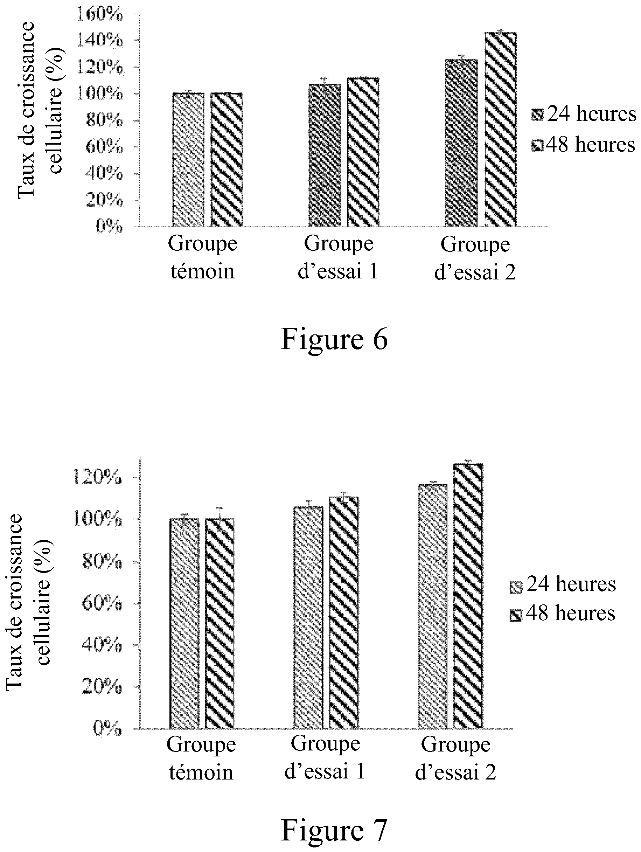

Les résultats des essais sont représentés à la Figure 6, à 24 h de culture, le taux de croissance cellulaire du groupe d'essai 1 par rapport au groupe témoin est de 107,2%, à 48 h de culture du groupe d'essai 1, le taux de croissance cellulaire par rapport au groupe témoin est de 111,6% ; à 24 h de culture, le taux de croissance cellulaire du groupe d'essai 2 par rapport au groupe témoin est de 125,7%, à 48 h de culture du groupe d'essai 2, le taux de croissance cellulaire par rapport au groupe témoin est de 145,9%.

### Essais de stimulation de croissance cellulaire des cellules de la papille dermique du follicule pileux humain par la Sarcodia suiae du mode de réalisation 3

Des extraits de Sarcodia suiae sont d'abord préparés selon le procédé au groupe d'essai 1, cependant la pression d'extraction est ajustée à 420 bars absolus pour préparer les extraits de Sarcodia suiae du mode de réalisation 3 ; parallèlement, des souches HFDPC de cellules de la papille dermique du follicule pileux humain sont préparées (acquises auprès de la société PromoCell, modèle de produit C12071) .

Une plaque de culture cellulaire est ensuite préparée, dans laquelle un liquide cellulaire contenant des cellules HFDPC est injecté à 9 puits, un groupe comprenant trois puits, les 9 puits auxquels le liquide cellulaire HFDPC susmentionné a été ajouté constituant le groupe témoin, le groupe d'essai 1 et le groupe d'essai 2, le groupe témoin ne contient pas d'extraits de Sarcodia suiae, une concentration finale de 5 µg/mL d'extraits de Sarcodia suiae est injectée au groupe d'essai 1 et une concentration finale de 25 µg/mL d'extraits de Sarcodia suiae est injectée au groupe d'essai 2. Une fois que des extraits de Sarcodia suiae ont été injectés au groupe 1 et au groupe 2, la plaque de culture cellulaire est placée dans un incubateur cellulaire pour culture dans un environnement à 37°C durant 48 heures, puis des échantillons sont prélevés de chaque puits à respectivement 24 h et 48 h de culture, les taux de croissance cellulaire sont mesurés par essai de taux de survie cellulaire (essai MTT).

Les résultats des essais sont représentés à la Figure 7. À 24 h de culture, le taux de croissance cellulaire du groupe d'essai 1 par rapport au groupe témoin est de 105,6%, à 48 h de culture du groupe d'essai 1, le taux de croissance cellulaire par rapport au groupe témoin est de 110,3% ; à 24 h de culture, le taux de croissance cellulaire du groupe d'essai 2 par rapport au groupe témoin est de 116,2%, à 48 h de culture du groupe d'essai 2, le taux de croissance cellulaire par rapport au groupe témoin est de 126,1%.

### Comparaison des empreintes HPLC de différents extraits de Sarcodia suiae du mode de réalisation 4

Des extraits de Sarcodia suiae A à E sont fournis comme échantillons de comparaison.

Les extraits de Sarcodia suiae A sont préparés selon le procédé d'extraction des extraits de Sarcodia suiae du mode de réalisation 1, et les conditions d'extraction sont les suivantes pour la préparation : les solvants d'extraction sont des fluides de dioxyde de carbone, la pression d'extraction est de 350 bars absolus, la température d'extraction est de 55°C et la durée d'extraction est de 6 heures.

Les extraits de Sarcodia suiae B sont préparés selon le procédé d'extraction des extraits de Sarcodia suiae du mode de réalisation 1, et les conditions d'extraction sont les suivantes pour la préparation : les solvants d'extraction sont des fluides de dioxyde de carbone, la pression d'extraction est de 650 bars absolus, la température d'extraction est de 55°C et la durée d'extraction est de 2 heures.

Les extraits de Sarcodia suiae C sont des extraits de Sarcodia suiae extraits à l'aide d'acétate d'éthyle, la température d'extraction est de 50°C, la - pression d'extraction est de 1 bar absolu et la durée d'extraction est de 6 heures.

Les extraits de Sarcodia suiae D sont des extraits de Sarcodia suiae extraits à l'aide d'eau pure, la température d'extraction est de 50°C, la pression d'extraction est de 1 bar absolu et la durée d'extraction est de 6 heures.

Les extraits de Sarcodia suiae E sont des extraits de Sarcodia suiae extraits à l'aide d'éthanol 95%, la température d'extraction est de 50°C, la pression d'extraction est de 1 bar absolu et la durée d'extraction est de 6 heures.

Une analyse HPLC des extraits de Sarcodia suiae A à E susmentionnés est ensuite effectuée selon les conditions suivantes : des colonnes HPLC TSKgel ODS-100S (250 × 4,6 mm) à débit 1 ml/min sont utilisées, une détection à l'aide d'un détecteur à ultraviolets/lumière visible est effectuée, la lumière détectée est celle d'UV de longueur d'onde de 280 nm.

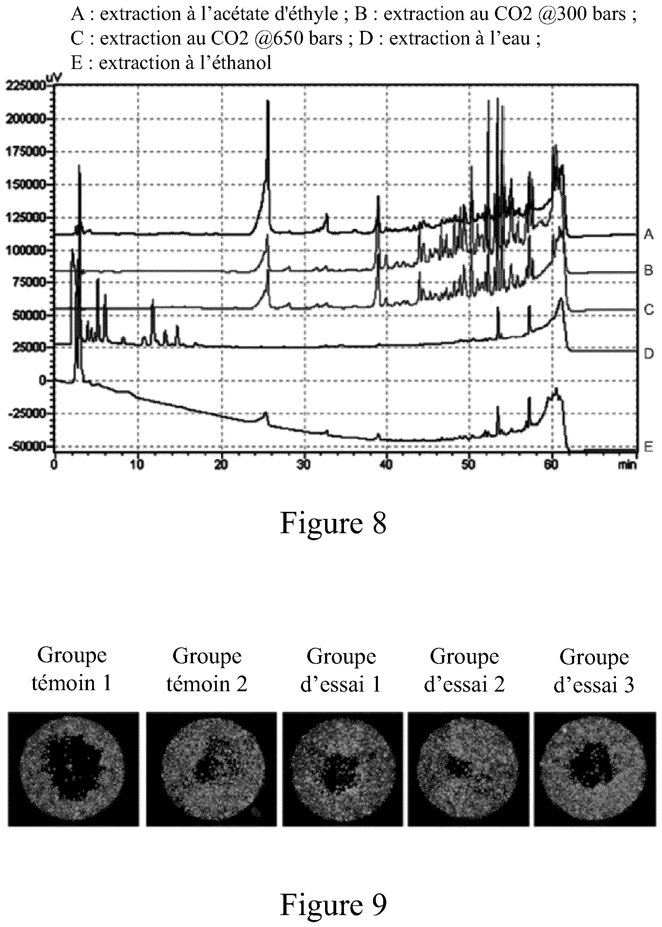

Les résultats des analyses sont représentés à la Figure 8. D'après les pics d'onde des empreintes HPLC de chacun des extraits de Sarcodia suiae de la Figure, le contenu des ingrédients des extraits de Sarcodia suiae A et B extraits selon différentes pressions d'extraction par fluides supercritiques est relativement proche, le contenu des ingrédients des extraits de Sarcodia suiae C à E extraits à l'aide des différents solvants acétate d'éthyle, eau et éthanol est différent de celui des extraits de Sarcodia suiae A et B.

### Essai d'inhibition de la croissance des bactéries de l'acné des extraits de Sarcodia suiae

Les extraits de Sarcodia suiae sont d'abord préparés selon le procédé d'extraction des extraits de Sarcodia suiae du mode de réalisation 1 ; parallèlement, les bactéries de l'acné sont préparées comme cibles de mesure des effets inhibiteurs par les extraits de Sarcodia suiae, les bactéries utilisées sont les Propionibacterium acnes (BCRC 10723). En outre, quatre plaques de milieu clostridial renforcé (RCM medium) sont préparées, le premier milieu de culture contient une concentration de 50 µg/mL de gentamycine et constitue le groupe témoin 1 ; aucun ingrédient supplémentaire n'a été ajouté au deuxième milieu de culture qui constitue le groupe témoin 2 ; une concentration de 1 mg/mL d'extraits de Sarcodia suiae susmentionnés a été ajoutée au troisième milieu de culture qui constitue le groupe d'essai 1 ; et une concentration de 0,5 mg/mL d'extraits de Sarcodia suiae susmentionnés a été ajoutée au troisième milieu de culture qui constitue le groupe d'essai 2.

Les bactéries de l'acné ont ensuite été inoculées au milieu clostridial renforcé (RCM), les quantités de bactéries de l'acné initialement inoculées étant de 1,15 × 10⁵ CFU/mL, après culture de 48 heures en milieu anaérobie à 37°C, un liquide bactérien de bactéries de l'acné est obtenu, et 100 µL de liquide bactérien de bactéries de l'acné ont été étalés de façon homogène sur les milieux de culture susmentionnés, et les milieux de culture susmentionnés sur lesquels du liquide bactérien de bactéries de l'acné ont été étalés ont été placés pour 48 heures de culture à 37°C dans un incubateur, une fois la culture achevée les milieux de culture susmentionnés sont sortis de l'incubateur, les unités de formation de colonies (CFU) des milieux de culture susmentionnés sont comptées.

Les résultats des essais du mode de réalisation 5 sont représentés au Tableau 2. Des antibiotiques ayant été ajoutés au groupe témoin 1, les effets bactéricides ont pu être atteints, lorsque la concentration d'extraits de Sarcodia suiae du mode de réalisation 5 atteint 0,5 mg/mL, par comparaison avec le groupe témoin 2, il y a phénomène d'inhibition de croissance bactérienne, lorsque la concentration d'extraits de Sarcodia suiae du mode de réalisation 5 atteint 1 mg/mL il y a effet bactéricide.

**Tableau 2 : Résultats des essais d'inhibition de croissance bactérienne des bactéries de l'acné par les extraits de Sarcodia suiae**

| Échantillons | Quantités de bactéries des milieux de culture (CFU/mL) |
|---|---|
| Groupe témoin 1 | 0 |
| Groupe témoin 2 | 6,77 × 10⁷ |
| Groupe d' essai 1 | 0 |
| Groupe d' essai 2 | 1,86 × 10⁴ |

### Essais de stimulation des capacités de migration des kératinocytes par les extraits de Sarcodia suiae du mode de réalisation 6

Des extraits de Sarcodia suiae F à H sont d'abord fournis comme échantillons de comparaison.

Les extraits de Sarcodia suiae sont préparés selon le procédé d'extraction des extraits de Sarcodia suiae du mode de réalisation 1, les conditions d'extraction sont respectivement fixées comme suit :
Extraits de Sarcodia suiae F : de la poudre fine d'algue de Sarcodia suiae séchée numéro 1081204 est utilisée comme cible de l'extraction, les solvants d'extraction utilisés sont des fluides de dioxyde de carbone, la pression d'extraction est de 365 bars absolus, la température d'extraction est de 55°C, durée d'extraction de 4 heures.

Extraits de Sarcodia suiae G : de la poudre fine d'algue de Sarcodia suiae séchée numéro 1081105 est utilisée comme cible de l'extraction, les solvants d'extraction utilisés sont des fluides de dioxyde de carbone, la pression d'extraction est de 365 bars absolus, la température d'extraction est de 55°C, durée d'extraction de 4 heures.

Extraits de Sarcodia suiae H : de la poudre fine d'algue de Sarcodia suiae séchée numéro 1081105-2 est utilisée comme cible de l'extraction, les solvants d'extraction utilisés sont des fluides de dioxyde de carbone, la pression d'extraction est de 420 bars absolus, la température d'extraction est de 55°C, durée d'extraction de 4 heures.

Une plaque de culture cellulaire à 96 puits et un kit d'essai Oris^{™} Cell Migration sont ensuite préparés, des bouchons (stopper) sont enfoncés dans 15 puits du kit d'essai Oris^{™} Cell Migration, une fois les bouchons en place dans les 15 puits susmentionnés, 2 × 10⁴ souches de kératinocytes humains (ATCC CRL-2309) sont respectivement inoculées dans lesdits puits susmentionnés, trois puits constituant un groupe, les 15 puits susmentionnés dans lesquels les souches de kératinocytes humains ont été inoculées étant respectivement divisés en groupe témoin 1, groupe témoin 2, groupe d'essai 1, groupe d'essai 2 et groupe d'essai 3. 0,05% de DMSO est ajouté au groupe témoin 1 ; 0,05% de DMSO et 100 ng/mL de facteur de croissance épidermique (EGF) (l'EGF stimulant la migration des kératinocytes humains) sont ajoutés au groupe témoin 2 ; 0,05% de DMSO et 1 µg/mL d'extraits de Sarcodia suiae F sont ajoutés au groupe d'essai 1 ; 0,05% de DMSO et 1 µg/mL d'extraits de Sarcodia suiae G sont ajoutés au groupe d'essai 2 ; 0,05% de DMSO et 1 µg/mL d'extraits de Sarcodia suiae H sont ajoutés au groupe d'essai 3. Une fois les échantillons à tester ajoutés à chacun des 5 groupes, la plaque de culture cellulaire à 96 puits susmentionnée est placée en incubateur cellulaire pour 24 heures de culture à 37°C, après 24 heures de culture, les bouchons de chaque groupe de puits sur la plaque de culture cellulaire à 96 puits susmentionnée sont retirés, la couche surnageante de chaque groupe de puits est éliminée, 50 µL d'agent fluorescent vital (Calcéine AM) (1 µM) sont ajoutés et le tout est laissé au repos pendant 1 heure.

Enfin, des images de 5 groupes de puits susmentionnées sont capturées au microscope à fluorescence Olympus CKX53, et les valeurs d'imagerie sont relevées, les valeurs des signaux fluorescents entourés sont utilisées au calcul des taux de migration cellulaire des souches de kératinocytes humains des puits des cinq groupes susmentionnés.

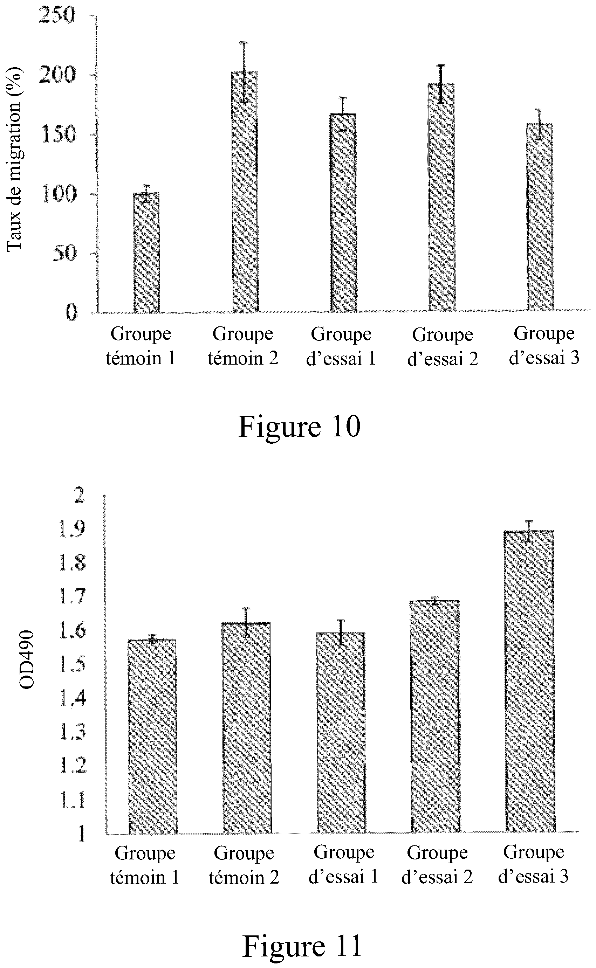

Les résultats des essais sont représentés à la Figure 9 et la Figure 10. Nous constatons d'après la Figure 9 que par comparaison avec le groupe témoin 1 auquel aucun ingrédient de stimulation de migration des kératinocytes humains n'a été ajouté, le groupe d'essai 1, le groupe d'essai 2 et le groupe d'essai 3 présentent une tendance manifeste à la migration cellulaire, en outre nous constatons d'après la Figure 10 que les taux de migration cellulaire du groupe d'essai 1, du groupe d'essai 2 et du groupe d'essai 3 sont tous supérieurs à celui du groupe témoin 1, le taux de migration du groupe témoin 1 est de 100%, le taux de migration du groupe d'essai 1 est de 165,78%, le taux de migration du groupe d'essai 2 est de 190,25% et le taux de migration du groupe d'essai 3 est de 156,08%.

### Essai de stimulation de croissance des kératinocytes humains par extraits de Sarcodia suiae du mode de réalisation 7

Les extraits de Sarcodia suiae sont préparés selon le procédé d'extraction des extraits de Sarcodia suiae du mode de réalisation 1 ; parallèlement, des souches de kératinocytes humains (ATCC CRL-2309) sont préparées comme cible du présent essai.

Une plaque de culture cellulaire à 96 puits est ensuite préparée, 2 × 10⁴ souches de kératinocytes humains (ATCC CRL-2309) sont respectivement inoculées dans 15 de ses puits, trois puits constituant un groupe, les 15 puits susmentionnés dans lesquels les souches de kératinocytes humains ont été inoculées étant respectivement divisés en groupe témoin 1, groupe témoin 2, groupe d'essai 1, groupe d'essai 2 et groupe d'essai 3. 1% de DMSO est ajouté au groupe témoin 1 ; 1% de DMSO et 100 ng/mL de facteur de croissance épidermique (EGF) sont ajoutés au groupe témoin 2 ; 1% de DMSO et 1,2 µg/mL d'extraits de Sarcodia suiae sont ajoutés au groupe d'essai 1 ; 1% de DMSO et 6,0 µg/mL d'extraits de Sarcodia suiae sont ajoutés au groupe d'essai 2 ; 1% de DMSO et 24,1 µg/mL d'extraits de Sarcodia suiae sont ajoutés au groupe d'essai 3.

Une fois les échantillons à tester ajoutés à chacun des 5 groupes, la plaque de culture cellulaire à 96 puits susmentionnée est placée en incubateur cellulaire pour 24 heures de culture à 37°C, après 24 heures de culture de la plaque de culture cellulaire à 96 puits, le taux de survie cellulaire est mesuré à l'aide d'un essai MTS/PMS. Le processus général de l'essai MTS/PMS du mode de réalisation 7 est le suivant : le réactif MTS/PMS est ajouté à la plaque de culture cellulaire à 96 puits pour 4 heures de réaction, la valeur d'absorption lumineuse à longueur d'onde 490 nm est mesurée et le taux de survie cellulaire est calculé.

Les résultats d'essai sont représentés à la Figure 11. La concentration de plus de 6,0 µg/mL d'extraits de Sarcodia suiae a des effets de stimulation de croissance des kératinocytes. La valeur d'absorption lumineuse OD₄₉₀ du groupe témoin 1 atteint 1,57, la valeur d'absorption lumineuse OD₄₉₀ du groupe témoin 2 atteint 1,62, la valeur d'absorption lumineuse OD₄₉₀ du groupe d'essai 1 atteint 1,59, la valeur d'absorption lumineuse OD₄₉₀ du groupe d'essai 2 atteint 1,68, la valeur d'absorption lumineuse OD₄₉₀ du groupe d'essai 3 atteint 1,89. Nous savons d'après les valeurs susmentionnées que le groupe d'essai 2 (contenant 6,0 µg/mL d'extraits de Sarcodia suiae) a des effets équivalents du groupe témoin 2 (ajout de facteur de croissance épidermique).

Le procédé d'extraits de Sarcodia suiae susmentionné fournit un nouveau procédé d'extraction pour extraire les ingrédients à haute valeur économique de la Sarcodia suiae, c'est-à-dire que les extraits de Sarcodia suiae obtenus grâce au procédé d'extraction susmentionné peuvent être utilisés dans les médicaments de stimulation de la croissance des cellules du follicule pileux, les médicaments ou produits cosmétiques inhibiteurs de croissance des bactéries de l'acné ou les médicaments ou produits cosmétiques de stimulation de l'activité des kératinocytes.

La présente invention est révélée dans le texte qui précède par des modes de réalisation préférés, la personne du métier doit comprendre que des modes de réalisation sont uniquement destinés à décrire la présente invention, et ne doivent pas être compris comme limitant l'étendue de la présente invention. Il faut noter que toute modification ou substitution équivalente desdits modes de réalisation est couverte par l'étendue de la présente invention. C'est pourquoi l'étendue de protection de la présente invention est définie par les limites de l'étendue des revendications.

## Revendications

1. - Procédé d'extraction de Sarcodia suiae, comprenant les étapes suivantes :
(a) fourniture d'une poudre séchée de Sarcodia suiae ;
(b) extraction de ladite poudre séchée de Sarcodia suiae par fluides de dioxyde de carbone afin d'obtenir le liquide d'extraction de Sarcodia suiae, ledit liquide d'extraction de Sarcodia suiae contenant des extraits de Sarcodia suiae et ledit solvant de fluides de dioxyde de carbone, la pression d'extraction étant de 300-650 bars absolus, la température d'extraction étant de 45-55°C, la durée d'extraction étant de 2-6 h ; et
(c) séparation des extraits de Sarcodia suiae dudit liquide d'extraction de Sarcodia suiae, la pression de séparation étant de 30-100 bars absolus.

2. - Procédé d'extraction de Sarcodia suiae selon la revendication 1, dans lequel la pression d'extraction est de 350 bars absolus, la température d'extraction est de 55°C, la durée d'extraction est de 2 h.

3. - Procédé d'extraction de Sarcodia suiae selon la revendication 1, dans lequel la pression d'extraction est de 365 bars absolus, la température d'extraction est de 55°C, la durée d'extraction est de 4 h.

4. - Procédé d'extraction de Sarcodia suiae selon la revendication 1, dans lequel la pression d'extraction est de 420 bars absolus, la température d'extraction est de 55°C, la durée d'extraction est de 4 h.

5. - Extraits de Sarcodia suiae, obtenus grâce audit procédé d'extraction selon l'une quelconque des revendications 1-4.

6. - Extraits de Sarcodia suiae selon la revendication 5 servant à la préparation de médicaments de stimulation de croissance du follicule pileux utilisant les extraits de Sarcodia suiae susmentionnés selon une concentration de plus de 5 µg/mL.

7. - Extraits de Sarcodia suiae selon la revendication 5 servant à la préparation de médicaments ou produits cosmétiques inhibiteurs de croissance des bactéries de l'acné utilisant les extraits de Sarcodia suiae susmentionnés selon une concentration de plus de 0,5 mg/mL.

8. - Extraits de Sarcodia suiae selon la revendication 5 servant à la préparation de médicaments ou produits cosmétiques renforçant l'activité de migration des kératinocytes utilisant les extraits de Sarcodia suiae susmentionnés selon une concentration de plus de 1 µg/mL.

9. - Extraits de Sarcodia suiae selon la revendication 5 servant à la préparation de médicaments ou produits cosmétiques renforçant l'activité de croissance des kératinocytes utilisant les extraits de Sarcodia suiae susmentionnés selon une concentration de plus de 6 µg/mL.
